# EUROPÄISCHE PATENTANMELDUNG

(11) **EP 3 443 993 A1**
(43) Veröffentlichungstag der Anmeldung: **20.02.2019**
(21) Anmeldenummer: 17186563.7
(22) Anmeldetag: 17.08.2017
(51) Int. Cl.: A61M 1/10, A61M 1/12

(54) **PUMPE MIT EINEM ROTORSENSOR ZUR ERFASSUNG VON PHYSIOLOGISCHEN PARAMETERN, STRÖMUNGS- UND BEWEGUNGSPARAMETERN**

(71) Anmelder: Berlin Heart GmbH, 12247 Berlin (DE)
(72) Erfinder: Kallenbach, Sebastian, 34128 Kassel (DE); Richert, Hendryk, 12487 Berlin (DE); Grasse, Martin, 12489 Berlin (DE); Krönert, Thaddäus, 14199 Berlin (DE); Peters, Oliver, 14129 Berlin (DE)
(74) Vertreter: Pfenning, Meinig & Partner mbB

(57) **Zusammenfassung**

Die Erfindung betrifft eine Pumpe (3) zur Förderung von Körperflüssigkeiten, insbesondere Blut, wobei die Pumpe (3) ein Pumpengehäuse (12) und einen im Pumpengehäuse (12) gelagerten Rotor (9) aufweist. Der Rotor (9) umfasst mindestens einen Sensor (15, 16, 17) zur Erfassung von Strömungs- und/oder Bewegungsparametern. Weiter betrifft die Erfindung ein Verfahren zum Betreiben der Pumpe (3).

## Beschreibung

Die Erfindung betrifft eine Pumpe zur Förderung von Körperflüssigkeiten, insbesondere Blut, wobei die Pumpe ein Pumpengehäuse und einen im Pumpengehäuse gelagerten Rotor aufweist.

Die Erfindung liegt auf dem Gebiet des Maschinenbaus, spezieller der Feinwerktechnik und der Elektrotechnik, und ist mit besonderem Vorteil im Bereich der Medizintechnik einsetzbar.

Insbesondere im medizinischen Bereich stellen Pumpen zur Förderung von Fluiden in einem Patientenkörper hohe technische Anforderungen. In diesem Zusammenhang sind beispielsweise Blutpumpen bekannt, die an einer ersten Stelle im Blutgefäßsystem eines Patienten Blut ansaugen und dieses zu einer zweiten Stelle innerhalb oder außerhalb des Blutgefäßsystems fördern. Typischerweise weisen die genannten Pumpen ein Pumpengehäuse und einen im Pumpengehäuse gelagerten Rotor auf. Der Rotor wird hierbei in der Regel durch eine im Pumpengehäuse vorgesehene Steuervorrichtung angesteuert. Mittels entsprechender Sensoren ist es heutzutage in einer Blutpumpe möglich, z.B. den Blutdruck, die Temperatur, die Beschleunigung des Pumpengehäuses und den Blutfluss innerhalb der Pumpe zu messen und zu schätzen. Basierend auf diesen Mess- bzw. Schätzwerten kann dann der Rotor entsprechend angesteuert werden. Es wird also ein Rückkopplungsmechanismus benutzt, um den Rotor möglichst präzise und bedarfsgemäß anzusteuern.

Die Veröffentlichung WO 2015/059158 A1 beschreibt z.B. ein Verfahren zum Betrieb einer derartigen Pumpeneinrichtung.

Es wäre wünschenswert, eine Pumpe zur Förderung von Körperflüssigkeiten zu entwickeln, bei der die Messung der Strömungskräfte und Turbulenz des Blutflusses zur Erkennung der Pumpenleistungsfähigkeit verbessert wird. Insbesondere wäre es wünschenswert, einen oder mehrere der folgenden Parameter besser vorhersagen oder erkennen zu können: Zustand der Pumpe, Thromben an dem Rotor und im Bereich der Pumpe, Zustand der Rotorlagerung und Pumpenlebensdauer.

Diese Aufgabe wird mittels einer Pumpe zur Förderung von Körperflüssigkeiten gemäß dem Hauptanspruch gelöst. Weiterbildungen sind Gegenstände der Unteransprüche sowie der nachfolgenden Beschreibung.

Dementsprechend bezieht sich die Erfindung auf eine Pumpe zur Förderung von Körperflüssigkeiten, insbesondere Blut, wobei die Pumpe ein Pumpengehäuse und einen im Pumpengehäuse gelagerten Rotor aufweist.

Der Rotor umfasst mindestens einen Sensor zur Erfassung von physiologischen Parametern und/oder Strömungs- und/oder Bewegungsparametern. Hierbei können die physiologischen Parameter, Strömungs- oder Bewegungsparameter beispielsweise Geschwindigkeit, Druck, Temperatur, Drehgeschwindigkeit, Winkelgeschwindigkeit, Beschleunigung, Sauerstoffgehalt, CO₂-Gehalt und/oder pH-Wert der Körperflüssigkeit; und/oder Position, Geschwindigkeit, Druck, Temperatur, Drehgeschwindigkeit, Betriebszustand, Winkelgeschwindigkeit und/oder Beschleunigung des Rotors und/oder der Pumpe sein. Der Sensor kann insbesondere dazu ausgestaltet sein, Änderungen oder Abweichungen der genannten Strömungs- oder Bewegungsparameter in Bezug auf vorbestimmte Werte, z.B. Sollwerte, zu erfassen. Der Sensor kann z.B. dazu ausgestaltet sein, Abweichungen einer Rotation des Rotors um eine Rotationsachse zu erfassen.

In einer Variante ist der Sensor z.B. ein Beschleunigungssensor, ein Gyroskop, ein Kraftsensor, ein Flusssensor, ein Sauerstoffsensor, ein CO₂-Sensor, ein Näherungssensor, ein Lichtsensor, ein Positionssensor, ein Drucksensor, ein Temperatursensor, ein Magnetfeldsensor (z.B. eine Spule) oder ein pH-Sensor. Der Sensor kann auch ein RFID-Sensor (radio-frequency identification sensor) sein. Der Rotor kann eine Vielzahl von Sensoren, z.B. 2, 3, 4, 5, 6 oder sogar mehr als 6 umfassen.

In einer Ausgestaltung der Erfindung ist der mindestens eine Sensor auf dem Rotor oder in dem Rotor oder innerhalb des Rotors angeordnet. Im Stand der Technik wurden die Sensoren bisher an dem statischen Pumpengehäuse angebracht. Beispielsweise ist im Stand der Technik eine Anordnung von Sensoren bei einem Rotor einer Blutpumpe vorgesehen, um eine Position des Rotors zu erfassen oder einen Betriebszustand des Rotors zu detektieren. Die Sensoren sind hier jedoch nicht auf dem Rotor oder in dem Rotor selbst montiert. Wegen des in der Regel vergleichsweise geringen Gewichts des Rotors in Bezug auf ein Gewicht des Pumpengehäuses kann der auf dem oder in dem Rotor angeordnete Sensor z.B. kleinste Abweichungen von Sollwerten erfassen. Daher kann mit der Pumpe gemäß dem Hauptanspruch ein empfindliches Messinstrument zur Verfügung gestellt werden.

Da der Rotor sich während des Betriebs der Pumpe dreht, ist eine Verkabelung des Rotors mit einer Energiequelle für den Sensor, die z.B. am Pumpengehäuse oder in einer externen, nicht im Patientenkörper angeordneten Vorrichtung vorgesehen ist, meistens unerwünscht.

Der Sensor selbst kann energieautark sein, d.h. der Sensor ist in diesem Fall selbst in der Lage Energie aus seinem Umfeld für seinen Betrieb zu nutzen. In diesem Fall kann der Sensor z.B. eine Sensorspule, ein piezoelektrisches Sensor-Element oder eine Sensor-Photodiode sein.

Alternativ oder zusätzlich kann der Rotor auch einen Energiespeicher, wie beispielsweise einen Kondensator, einen aufladbaren Akku oder eine Batterie für eine Energieversorgung des Sensors aufweisen.

In einer weiteren Ausgestaltung weist der Rotor einen Energiewandler (auch Transducer genannt) zur drahtlosen Energieversorgung des Sensors auf. Drahtlos heißt im Sinne der vorliegenden Schrift, dass der Sensor und/oder der Rotor nicht mit statischen, nicht beweglichen Bauteilen des Pumpengehäuses verdrahtet sind und dass der Sensor in der Regel lediglich mit Komponenten auf dem Rotor verdrahtet ist. Hierdurch kann sich der Rotor während des Pumpenbetriebs ungestört drehen und ein Verschleiß der Bauteile wird verringert. Der Energiewandler ist insbesondere dazu ausgestaltet, Energie einer nicht mit dem Rotor verbundenen Energiequelle in eine für den Sensor nutzbare Energieform umzuwandeln.

Der oben genannte Energiespeicher kann insbesondere durch in dem Energiewandler erzeugten elektrischen Strom aufladbar sein. Der Energiespeicher kann mit dem mindestens einen Sensor zu dessen Stromversorgung verbunden sein.

Die Pumpe kann weiter eine Energiequelle für den Sensor aufweisen. Beispielsweise ist die Energiequelle dazu ausgestaltet, Energie drahtlos an den Energiewandler zu übertragen. Die Energiequelle und der Energiewandler sind typischerweise derart ausgestaltet, dass von der Energiequelle bereitgestellte Energie im Energiewandler des Rotors umwandelbar ist. Die durch die Energiequelle bereitgestellte Energie wird dann in der Regel durch den Energiewandler in eine für den Betrieb des Sensors notwendige Energieform umgewandelt, z.B. in elektrischen Strom. Die Energiequelle kann also energetisch mit dem Energiewandler gekoppelt sein. Die Energiequelle kann z.B. in dem Pumpengehäuse vorgesehen sein. Weiter ist die Energiequelle üblicherweise nicht elektrisch oder mechanisch mit dem Rotor verbunden. Die Energiequelle muss nicht notwendigerweise ein Bestandteil des Pumpengehäuses oder der Pumpe sein, sie kann z.B. in einer separaten, innerhalb oder außerhalb des Patientenkörpers angeordneten Vorrichtung vorgesehen sein oder eine separate Vorrichtung bilden.

Der Energiewandler kann z.B. eine Rotorspule, ein piezoelektrischer Rotorwandler oder eine Photodiode sein. Für eine energetische Kopplung mit der Rotorspule, der Photodiode oder dem piezoelektrischen Rotorwandler kann die Energiequelle dann eine Statorspule, eine Lichtquelle bzw. eine Schallquelle sein. Es können gleichzeitig mehrere gleiche oder verschiedenartige Energiequellen und mehrere gleiche oder verschiedenartige Energiewandler vorgesehen sein.

Die Rotorspule kann derart ausgebildet sein, dass in der Rotorspule bei einer Rotordrehung eine Spannung induzierbar ist. Typischerweise ist die Rotorspule also ausgebildet, bei einer Rotordrehung einen elektrischen Strom für den Betrieb des mindestens einen Sensors bereitzustellen. Dies erfolgt z.B. durch eine zeitlich veränderliche Magnetfeldvariation (z. B. durch Motorrastmomente) in der Rotorspule. Zu diesem Zweck kann es vorgesehen sein, dass das Pumpengehäuse für die Induktion der Spannung in der Rotorspule im Bereich des Rotors mindestens einen Permanentmagneten aufweist. Durch die Drehung des Rotors und der Rotorspule in Bezug auf das Pumpengehäuse und den Permanentmagneten wird also eine Spannung in der Rotorspule induziert. Der Permanentmagnet oder die Permanentmagneten ist/sind vorzugsweise derart im Pumpengehäuse angeordnet, dass bei einer vorgegebenen Drehgeschwindigkeit des Rotors der Permanentmagnet oder die Permanentmagneten einen vorbestimmten Spannungsverlauf in der sich mit drehenden Rotorspule induzieren. Eine Frequenz der in der Rotorspule induzierten Wechselspannung hängt von der Anzahl der Permanentmagneten sowie von der Drehgeschwindigkeit des Rotors ab.

Alternativ oder zusätzlich kann es vorgesehen sein, dass das Pumpengehäuse eine Statorspule als Energiequelle für den Sensor aufweist, wobei eine vorbestimmte Spannung bzw. ein vorbestimmter zeitlicher Spannungsverlauf in der Rotorspule durch eine Bestromung der Statorspule induzierbar ist. Typischerweise sind in aktiv magnetisch gelagerten Blutpumpen zumindest im Rotor Permanentmagneten integriert, während sich im Pumpengehäuse Permanentmagneten und Spulen mit magnetischen Rückschlüssen befinden. An diesen Spulen im Pumpengehäuse wird also für die Lagerung und Rotation des Rotors eine (Wechsel)Spannung angelegt. Diese Spannung kann nun gemäß der vorstehend beschriebenen Ausbildung mit einer vorbestimmten Spannung moduliert werden, damit eine vorbestimmte Spannung in der Rotorspule induziert wird. Mittels der Rotorspule und vorzugsweise der Statorspule kann also drahtlos Energie übertragen werden. Hierzu ist es vorteilhaft, wenn die Statorspule den Rotor zumindest bereichsweise umschließt. Eine Frequenz der in der Rotorspule induzierten Spannung kann sich von einer Drehfrequenz des Rotors unterscheiden. Z.B. kann die Frequenz der in der Rotorspule induzierten Wechselspannung kleiner oder größer als die Drehfrequenz des Rotors. Die Frequenz der in der Rotorspule induzierten Spannung kann z.B. größer als 100 Hz, größer als 150 Hz oder größer als 200 Hz sein.

Es sei angemerkt, dass Abmessungen und Induktivität der Rotorspule sich typischerweise nach dem Aufbau der Energie-Übertragungsstrecke richten.

In einer weiteren Ausbildung ist der Energiewandler ein piezoelektrischer Rotorwandler. Der piezoelektrische Rotorwandler ist dazu ausgebildet, Energie einer mechanischen Druckwelle, wie z.B. einer Schallwelle oder einer Ultraschallwelle, in elektrischen Strom umzuwandeln. Die mechanischen Druckwellen werden typischerweise durch den piezoelektrischen Wandler aufgenommen, wodurch sich ein piezoelektrischer Kristall im piezoelektrischen Wandler verformt. Die genannte Verformung produziert den elektrischen Strom, der durch den Sensor verwendet wird. Ein Vorteil von piezoelektrischen Wandlern gegenüber Spulen ist, dass die mechanischen Druckwellen sich im Körper eines Patienten besonders gut ausbreiten können. Sie werden insbesondere nicht durch ein metallisches Pumpengehäuse abgeschirmt. Hierdurch kann man den Ort des piezoelektrischen Wandlers auf dem Rotor oder in dem Rotor relativ flexibel wählen. Weiter besteht Flexibilität bei der Wahl der Materialien, z.B. des Gehäuses und des Rotors, und bei der Wahl des Orts der Energiequelle. Die Energiequelle für den piezoelektrischen Rotorwandler kann z.B. eine Schallquelle, insbesondere eine Ultraschalquelle, sein, die im Pumpengehäuse oder außerhalb des Pumpengehäuses angeordnet ist. Die Schallquelle kann beispielsweise ein Lautsprecher oder ein piezoelektrisches Element sein. Die Schallquelle kann stationär oder gepulst betreibbar sein. In einer weiteren Ausgestaltung weist die Pumpe einen piezoelektrischen Pumpenwandler für eine piezoelektrische Kopplung mit dem piezoelektrischen Rotorwandler auf. Prinzipiell kann z.B. auch eine Lichtquelle als Energiequelle und eine auf dem Rotor vorgesehene Photodiode oder Solarzelle als Energiewandler vorgesehen sein. Die Wahl einer von der Lichtquelle zu emittierenden Wellenlänge hängt von dem Absorptionsverhalten der Körperflüssigkeit und der Effizienz der Photodiode oder Solarzelle ab. Die Lichtquelle und die Photodiode oder Solarzelle sollten dazu ausgestaltet sein, für Körperflüssigkeiten, insbesondere Blut, durchlässiges Licht auszusenden bzw. zu absorbieren und in eine für den Sensor verwendbare Energieform umzuwandeln. Eine geeignete Wellenlänge des von der Lichtquelle emittierten Lichts kann zum Beispiel mindestens 600 nm, vorzugsweise mindestens 650 nm, insbesondere mindestens 700 nm betragen. Die Wellenlänge des von der Lichtquelle emittierten Lichts kann maximal 1500 nm, oder maximal 1300 nm betragen. Bei größeren Wellenlängen kann die Effizienz von üblichen Photodioden oder Solarzellen rasch abnehmen. Als Lichtquelle kommen z.B. eine LED, ein Laser oder eine Leuchte in Betracht. Die Lichtquelle kann stationär oder gepulst betreibbar sein.

Typischerweise umfasst die Pumpe eine drahtlose Kommunikationsschnittstelle zwischen dem Sensor und mindestens einer weiteren Komponenten der Pumpe oder einer Pumpeneinrichtung (s. unten), wie z.B. einer Steuer- und Verarbeitungseinheit (s. unten). Hierdurch können Daten oder Signale des Sensors drahtlos an die mindestens eine weitere Komponente übermittelt werden. Die drahtlose Kommunikation der Kommunikationsschnittstelle kann z.B. über Funksignale, eine induktive Kupplung, optische Signale, akustische Signale oder andere geeignete Signale erfolgen. Die Kommunikationsschnittstelle kann z.B. eine oder mehrere Kommunikationseinheiten aufweisen.

In einer Ausgestaltung umfasst die Pumpe eine erste Kommunikationseinheit zum drahtlosen Übermitteln von Sensorsignalen des Sensors. Die erste Kommunikationseinheit kann auf oder in dem Rotor angeordnet sein. In einer Variante ist der Energiewandler gleichzeitig als erste Kommunikationseinheit ausgestaltet. Hierdurch kann die Anzahl der zu verbauenden Komponenten gering gehalten werden. Es kann alternativ vorgesehen sein, dass die erste Kommunikationseinheit sich von dem Energiewandler unterscheidet. Zur Übermittlung der Sensorsignale kann die erste Kommunikationseinheit eine entsprechende Sendeeinheit zum Übermitteln von Signalen, wie eine Antenne, eine Lichtquelle, eine Spule oder eine Schallquelle, aufweisen. Optional kann die erste Kommunikationseinheit auf dem Rotor zusätzlich eine Empfangseinheit zum Empfangen von Signalen aufweisen. Beispielsweise umfasst die erste Kommunikationseinheit z.B. eine Antenne zum Versenden und/oder Empfangen von Funksignalen. Die erste Kommunikationseinheit kann die Daten und/oder Signale der Sensoren typischerweise in Abständen von 10 ms bis 100 ms an eine zweite Kommunikationseinheit (s. unten) übermitteln.

Je nach Ausführung der Pumpe kann das Pumpengehäuse eine Auslesevorrichtung zum drahtlosen Auslesen des mindestens einen Sensors aufweisen. In diesem Fall kann auf die vorstehend genannte erste Kommunikationseinheit verzichtet werden.

Weiter wird mit der Erfindung eine Pumpeneinrichtung vorgeschlagen. Die Pumpeneinrichtung umfasst eine zuvor genannte Pumpe und eine Steuer- und Verarbeitungseinheit. Die Steuer- und Verarbeitungseinheit ist vorzugsweise ausgestaltet, Sensorsignale des mindestens einen Sensors auszuwerten und basierend auf den Sensorsignalen die Pumpe anzusteuern. Um ein möglichst kompaktes System zu schaffen, kann die Steuer- und Verarbeitungseinheit im Pumpengehäuse vorgesehen sein. Sie kann aber auch als separate Einheit vorgesehen sein. Die Pumpe kann z.B. in einem Körper eines Patienten implantierbar bzw. implantiert sein. Die Steuer- und Verarbeitungseinheit kann sich in einer Ausgestaltung innerhalb oder außerhalb des Körpers des Patienten befinden. Alternativ ist, wie bereits beschrieben, die Steuer- und Verarbeitungseinheit integraler Bestandteil der implantierten Pumpe.

Die Steuer- und Verarbeitungseinheit kann eine zweite Kommunikationseinheit zur drahtlosen Kommunikation mit der ersten Kommunikationseinheit aufweisen. Hierzu ist ein entsprechender Kommunikationskanal oder eine Kommunikationsschnittstelle zwischen den beiden Kommunikationseinheiten vorgesehen. Die erste Kommunikationseinheit ist vorzugsweise ausgestaltet, die Sensordaten und/oder Sensorsignale an die zweite Kommunikationseinheit zu übermitteln. Die zweite Kommunikationseinheit ist vorzugsweise dazu ausgestaltet, die Sensordaten und/oder die Sensorsignale zu empfangen. In einer weiteren Ausgestaltung ist die zweite Kommunikationseinheit dazu konfiguriert, Daten an die erste Kommunikationseinheit zu übermitteln und die erste Kommunikationseinheit ist dazu konfiguriert die Daten von der zweiten Kommunikationseinheit zu empfangen.

Falls die oben genannte Auslesevorrichtung vorgesehen ist, kann diese zur Übertragung der Sensorsignale mit der Steuer- und Verarbeitungseinheit verbunden sein.

Die Steuer- und Verarbeitungseinheit kann dazu ausgelegt sein, Signale oder Daten des oben genannten Sensors oder mehrerer der oben genannten Sensoren auszuwerten oder zu verarbeiten. Für die Verarbeitung und/oder Auswertung der Signale und/oder der Daten der oben genannten Sensoren kann die Steuer- und Verarbeitungseinheit einen Microcontroller, einen Prozessor, einen Mikroprozessor und/oder einen digitalen Signalprozessor aufweisen. Hierbei kann ein digitaler Signalprozessor (DSP) zu einer kontinuierlichen Bearbeitung von digitalen Signalen ausgestaltet sein, beispielsweise digitalen Signalen eines oder mehrerer der oben genannten Sensoren.

Weiterhin kann die Steuer- und Verarbeitungseinheit einen oder mehrere Speicher aufweisen, wie z.B. random access memory (RAM), read only memory (ROM), eine Festplatte, ein magnetisches Speichermedium und/oder ein optisches Laufwerk. Im Speicher kann ein Programm gespeichert sein, z.B. eine Software zur Verarbeitung oder Bearbeitung der Daten und/oder der Signale eines Sensors oder mehrerer der oben genannten Sensoren.

Die Steuer- und Verarbeitungseinheit kann außerdem ausgebildet sein, basierend auf oder abhängig von den Sensordaten oder Sensorsignalen die Pumpe und/oder die Energiequelle und/oder den mindestens einen Sensor und/oder den Rotor und/oder andere Geräte anzusteuern und/oder eine bestimmte Aktion auszuführen.

Die Steuer- und Verarbeitungseinheit kann z.B. dazu ausgebildet sein, die Energiequelle, wie z.B. die Statorspule, derart anzusteuern, dass eine vorbestimmte Energiemenge oder Leistung durch den Energiewandler, wie z.B. die Rotorspule, aufgenommen wird, und der Energiewandler eine vorbestimmte Energiemenge oder Leistung an den Sensor liefert.

In einer Variante kann die Rotorspule gleichzeitig als Energiewandler und als Kommunikationseinheit ausgebildet sein. Die Rotorspule übernimmt in diesem Fall sowohl die Energieübertragung als auch die Datenübertragung.

Die Pumpe kann eine Kodierungseinheit umfassen, welche dazu ausgebildet ist, Signale des Sensors zu kodieren und die kodierten Signale an die Kommunikationseinheit, wie die Rotorspule, weiterzuleiten. Die Kodierungseinheit befindet sich typischerweise ebenfalls auf dem Rotor oder in dem Rotor.

Es kann in einer weiteren Ausführungsform vorgesehen sein, dass die Rotorspule gleichzeitig als Sensor und erste Kommunikationseinheit ausgebildet ist. In diesem Fall kann auf einen zusätzlichen Energiewandler verzichtet werden. Eine zweite an dem Pumpengehäuse angeordnete Spule kann dann durch die Rotorspule induzierte Magnetfeldänderungen messen und an die Steuer- und Verarbeitungseinheit weiterleiten.

Das Pumpengehäuse kann als Kunststoffgehäuse, Keramikgehäuse oder Metallgehäuse ausgebildet sein. Das Pumpengehäuse kann auch eine Kombination der zuvor genannten Materialien (Kunststoff, Keramik und/oder Metall) enthalten. Die Materialwahl für das Pumpengehäuse kann durch die Wahl des Sensors, des Energiewandlers, der Energiequelle und der ersten Kommunikationseinheit beeinflusst sein.

Typischerweise beträgt ein Gewicht der Pumpe insgesamt mindestens 50 g und/oder höchstens 250 g. Das Gewicht des Rotors einer Axialpumpe beträgt höchstens 25 g, vorzugsweise höchstens 15 g. Weiterhin beträgt das Gewicht des Rotors einer Radialpumpe höchstens 40 g, vorzugsweise höchstens 30 g. Weiterhin kann der Rotor aktiv magnetisch oder mechanisch oder hydrodynamisch gelagert sein.

Mit der Erfindung wird auch ein Verfahren zum Betreiben der zuvor beschriebenen Pumpe zur Förderung von Körperflüssigkeiten, insbesondere Blut, wobei die Pumpe ein Pumpengehäuse und einen im Pumpengehäuse gelagerten Rotor aufweist.

Das Verfahren enthält zumindest den Schritt: Erfassen von physiologischen Parametern und/oder Strömungs- und/oder Bewegungsparametern durch einen Sensor, der auf dem Rotor oder in dem Rotor angeordnet ist.

Es sei an dieser Stelle betont, dass die im Zusammenhang mit der Pumpe oder der Pumpeneinrichtung beschriebenen Merkmale auch für das Verfahren beansprucht werden können.

Im Folgenden wird die Erfindung anhand beigefügter Figuren weiter erläutert.

Dabei zeigen
- Fig. 1: schematisch die Darstellung eines Patientenkörpers mit einer Herzkatheterpumpe, die über die Aorta in eine Herzkammer eingeführt ist;
- Fig. 2: in vergrößerter Darstellung einen durch einen Aortabogen eingeführten Herzkatheter mit einer Herzpumpe;
- Fig. 3: eine Ansicht eines Axialrotors;
- Fig. 4: eine schematische Darstellung einer Rotorschaltung;
- Fig. 5: eine weitere schematische Darstellung einer Rotorschaltung gemäß der Erfindung.

Nachfolgend sind wiederkehrende Elemente mit den gleichen Bezugszeichen versehen.

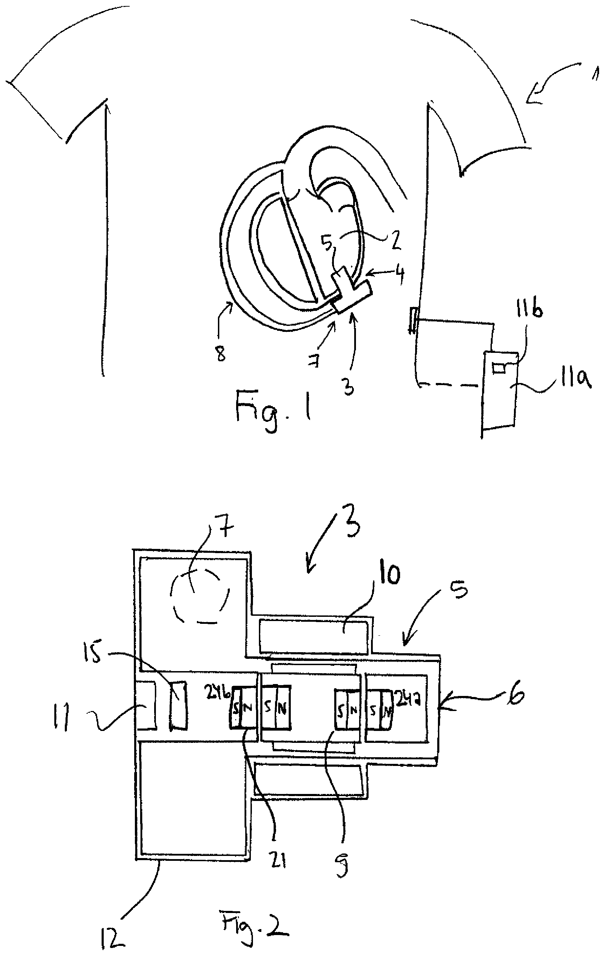

Fig. 1 zeigt schematisch einen Patientenkörper 1 mit einer Herzkammer 2, in welche eine Blutpumpe 3 eingeführt. Die Blutpumpe 3 ist dabei mit dem Apex 4 der Herzkammer verbunden. Im gezeigten Beispiel ist die Pumpe 3 mit dem linken Ventrikel des Herzens verbunden, d.h. die Pumpe 3 ist eine Left Ventricular Assist Device oder auch LVAD.

In der Fig. 2 ist die Blutpumpe 3 vergrößert dargestellt. Ein Einlassstutzen 5 der Blutpumpe 3, welche direkt in den Apex 4 eingeführt ist, weist im vorliegenden Ausführungsbeispiel eine Ansaugöffnung 6 auf, durch welche das Blut eingesaugt wird. Das Blut wird im vorliegenden Ausführungsbeispiel mittels einer axialen Blutpumpe 3 mit einem tangentialen Auslass 7 gefördert. Der Auslass 7 ist mittels einer Kanüle 8 oder eines schlauchartigen Objekts mit der Aorta verbunden. Alternativ kann der Auslass 7 mit der Pulmonalarterie (bei einer RVAD) oder der Schulterarterie verbunden werden. Im Einlassstutzen 5 befindet sich ein mit einer Wendel 14 versehener Rotor 9, welcher über einen Stator 10 der Blutpumpe 3 in Rotation versetzt werden kann und so das Blut in die Ansaugöffnungen 6 saugt. Ein Beispiel einer derartigen Blutpumpe ist der WO 2012/149946 A1, WO 2011/054545 A1 oder der WO 2012/150045 A1 oder in der WO 2015/059158 A1 entnehmbar, deren Offenbarungen durch Referenzieren vollumfänglich in der vorliegenden Anmeldung aufgenommen sind.

Weitere Beispiele geeigneter Blutpumpen sind die Blutpumpen der EXCOR® bzw. INCOR® Baureihe der Berlin Heart GmbH Berlin, Deutschland. Bei der Verwendung dieser Blutpumpen werden die Pumpen über Kanülen mit dem Herzen verbunden, so dass das Ansaugelement sowohl der Pumpe selbst als auch der Einlass der Kanüle, welche die Pumpe mit dem Herzen verbindet, sein kann.

Die Blutpumpe 3 umfasst eine Steuervorrichtung 11, 11b oder 31 zur Steuerung des Rotors 9, welche beispielsweise innerhalb eines Pumpengehäuses 12 oder in einem separaten, zusätzlichen innerhalb oder außerhalb des Körpers angeordneten Gehäuses 11a angeordnet sein kann. Die Steuerung erfolgt üblicherweise mittels einer automatischen Rückkopplungsschleife.

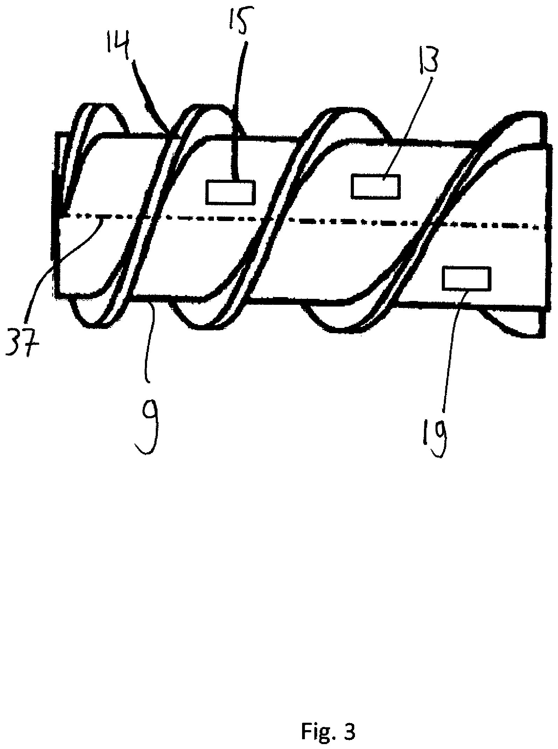

Die Fig. 3 zeigt eine Ansicht des Rotors 9 mit der Wendel 14. Der Rotor 9 der Fig. 3 kann insbesondere in der Pumpe 3 der Fign. 2 und 1 eingesetzt werden. Der im Pumpengehäuse 12 gelagerte Rotor 9 ist aktiv-magnetisch gelagert und weist mindestens einen Sensor 15 zur Erfassung von Strömungs- und Bewegungsparametern auf. Im Rotor 9 befinden sich Neodym-Eisen-Bor Magneten, während sich im Pumpengehäuse 12 passive Magneten 21 und aktive Magnetspulen im Stator 10 für den Antrieb und die Axial-Radial Lagerung des Rotors 9 befinden. Der Rotor 9 bildet das einzige bewegliche Teil in der Pumpe 3.

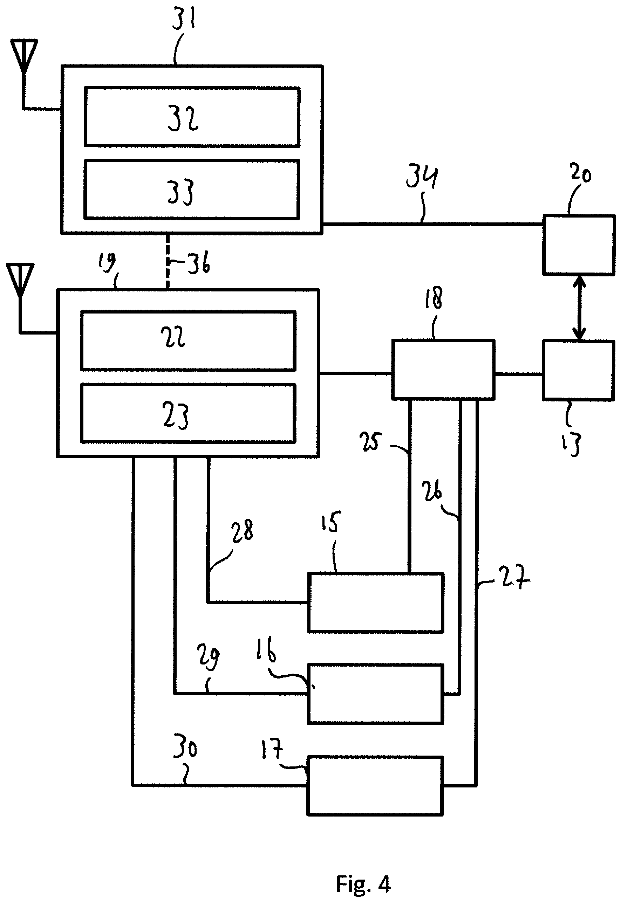

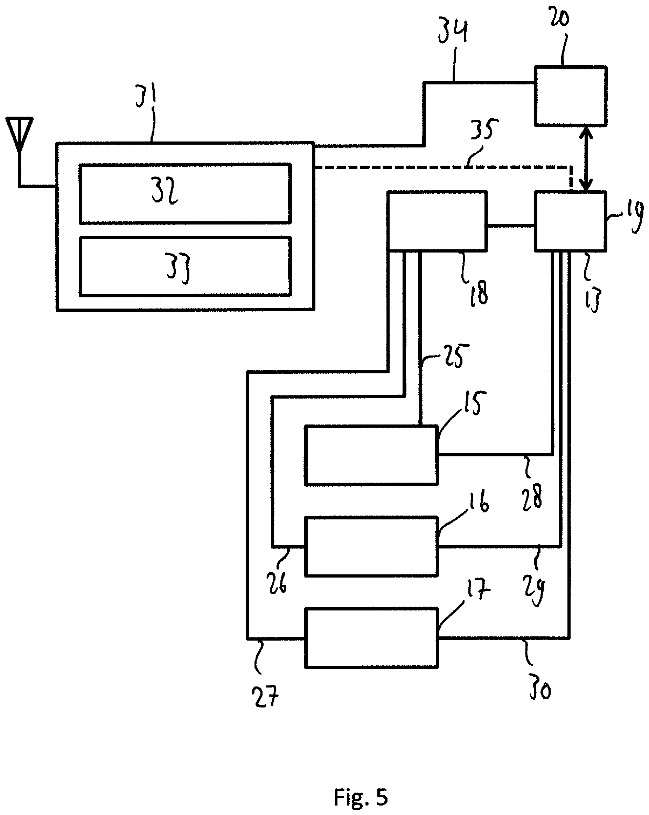

Der Rotor 9 weist mindestens einen Sensor 15 auf. Wie in den Fign. 4 und 5 angedeutet, können auch mehrere Sensoren 15, 16 und 17 auf oder in dem Rotor 9 vorgesehen sein. Durch Auswertung der Sensorsignale können Aussagen über den Zustand der Pumpe 3, über entstehende Thromben an der Wendel 14 des Rotors 9 oder im Pumpenbereich und über den Zustand der Lagerung des Rotors 9 getroffen werden. Weiter kann hierdurch eine Vorhersage der Pumpenlebensdauer getroffen werden. Hierzu können die Sensoren 15, 16, 17 ausgestaltet sein, Bewegungs- oder Strömungsparameter zu erfassen. Beispielsweise können die Sensoren 15, 16, 17 dazu ausgestaltet sein, Abweichungen einer Rotation des Rotors 9 um eine Rotationsachse 37 zu erfassen.

Als Sensor 15 bzw. Sensoren 15, 16, 17 kommen verschiedene Sensoren in Betracht. Beispielsweise kann der Sensor 15 oder die Sensoren 15, 16, 17 ein Beschleunigungssensor, ein Gyroskop, ein Kraftsensor, ein Flusssensor, ein Sauerstoffsensor, ein CO2-Sensor, ein Näherungssensor, ein Lichtsensor, ein Positionssensor, ein Drucksensor, ein Temperatursensor, ein Magnetfeldsensor, eine Spule, ein RFID-Sensor oder ein pH-Sensor sein. Es können auch mehrere verschiedenartige Sensoren 15, 16, 17 vorgesehen sein. Der Sensor 15, 16, 17 kann beispielsweise als Spule ausgebildet sein. Der mindestens eine Sensor 15 ist mit einer ersten Kommunikationseinheit 19 verbunden, auf die weiter unten eingegangen wird.

Zur Energieversorgung des Sensors 15 oder der Sensoren 15, 16, 17 umfasst der Rotor 9 einen oder mehrere Energiewandler 13 (manchmal auch Transducer genannt). Der Energiewandler 13 kann z.B. als Rotorspule, Photodiode/Solarzelle oder als piezoelektrischer Wandler ausgebildet sein. Mittels des auf dem Rotor 9 angeordneten Energiewandlers 13 kann also der Sensor 15 bzw. die Sensoren 15, 16, 17 drahtlos seine Energie von einer nicht auf dem Rotor 9 angeordneten Energiequelle 20 beziehen. Um eine Energieversorgung der Sensoren 15, 16, 17 zu verbessern bzw. eine ständige Energieversorgung zu gewährleisten, kann der Rotor 9 einen Akku 18 zur Stromversorgung der Sensoren 15, 16, 17 aufweisen, wobei der Akku 18 durch in dem Energiewandler 13 erzeugten elektrischen Strom aufladbar ist. Alternativ zum Energiewandler 13 kann auch eine Batterie oder ein aufladbarer Akku für eine Energieversorgung des Sensors 15, 16 17 auf oder in dem Rotor 9 angeordnet sein.

Bei einer Ausbildung des Energiewandlers 13 als Rotorspule können das Pumpengehäuse 12 und die Rotorspule derart ausgebildet sein, dass in der Rotorspule bei einer Rotordrehung eine Spannung induzierbar ist. Auf der Seite des Pumpengehäuses 12 kann dies z.B. durch einen passiven Permanentmagneten bewerkstelligt werden, es kann aber auch eine aktive Statorspule vorgesehen sein, die ausgebildet ist, eine vorbestimmte Spannung in der Rotorspule zu induzieren, um den Energiebedarf des Sensors 15, 16, 17 zu decken. Die Statorspule kann im Stator 10 angeordnet sein und den Rotor 9 vollständig umschließen.

Bei einer Ausbildung des Energiewandlers 13 als piezoelektrischer Pumpenwandler ist dieser ausgebildet, mechanische Druckwellen, insbesondere Ultraschallwellen, in elektrische Energie umzusetzen. Wenn eine mechanische Druckwelle auf den piezoelektrischen Wandler 13 trifft, verformt sich in dem Wandler ein piezoelektrischer Kristall, wodurch ein Strom in die piezoelektrischen Wandler generiert wird. Für die Bereitstellung der mechanischen Druckwellen kann vorzugsweise in dem Pumpengehäuse 12 ein weiterer piezoelektrischer Wandler oder eine andersartige Schallquelle vorgesehen sein.

Der Energiewandler 13 kann alternativ oder zusätzlich auch eine Solarzelle oder Photodiode sein, welche Lichtenergie in elektrische Energie umwandelt.

Die Fig. 4 zeigt ein Beispiel einer Verschaltung der Baugruppen, die sich auf dem und in dem Rotor 9 sowie in dem Pumpengehäuse 12 befinden. In der Fig. 4 ist eine Energiequelle 20 gezeigt, welche Energie für den Energiewandler 13 bereitstellt. Hierbei ist die Energiequelle 20 vorzugsweise am statischen Pumpengehäuse 12 angeordnet, während der Energiewandler 13 auf oder in dem Rotor 9 vorgesehen ist. Der Energiewandler 13 ist mit einem Akkumulator 18 und/oder einem Gleichrichter 18 verbunden. Der Akkumulator 18 bzw. der Gleichrichter 18 sorgt dafür, dass die Sensoren 15, 16 und 17 mit einer möglichst konstanten Gleichspannung/Gleichstrom versorgt werden können. Hierzu ist der Akkumulator 18 bzw. der Gleichrichter 18 über Versorgungsleitungen 25, 26 und 27 mit den Sensoren 15, 16 bzw. 17 verbunden. Die Sensoren 15, 16, 17 sind mittels Kommunikationsleitungen 28, 29 bzw. 30 mit der ersten Kommunikationseinheit 19 verbunden. Die erste Kommunikationseinheit 19 befindet sich auch auf dem Rotor 9 oder innerhalb des Rotors 9. Die erste Kommunikationseinheit 19 weist ein Sende- und Empfangsmodul 22 sowie einen Digital-/Analogwandler 23 auf. Die Sensorsignale werden in dem Digital-/Analogwandler in digitale Signale umgesetzt oder kodiert und an das Sende- und Empfangsmodul 22 weitergeleitet. Das Sende- und Empfangsmodul 22 wandelt die digitalen Signale der Sensoren in zu übertragende Daten um und sendet die zu übertragenden Daten drahtlos über eine Antenne zu einer zweiten Kommunikationseinheit 33.

Weiter ist in der Fig. 4 eine Steuer- und Verarbeitungseinheit 31 gezeigt, welche identisch mit der Steuervorrichtung 11 der Fign. 1 und 2 sein kann. Die Steuer- und Verarbeitungseinheit 31 weist z.B. eine Rotorsteuerung und eine zweite Kommunikationseinheit 33 auf. Die zweite Kommunikationseinheit 33 ist drahtlos mit der ersten Kommunikationseinheit 19 über Kommunikationskanal 36 verbunden. Obwohl in der Fig. 4 die Kommunikationseinheit 19 und die zweite Kommunikationseinheit 33 jeweils eine Antenne aufweisen, können für die drahtlose Kommunikation zwischen den beiden Kommunikationseinheiten 19 und 33 auch andere Mittel vorgesehen sein. So können in jeder der beiden Kommunikationseinheiten 19 und 33 eine Spule oder ein piezoelektrischer Wandler für die Kommunikation zwischen den beiden Kommunikationseinheiten 19 und 33 vorgesehen sein.

Die Ausführungsform der Fig. 5 unterscheidet sich von der Ausführungsform der Fig. 4 dahingehend, dass der Energiewandler 13 der Fig. 5 außerdem als Kommunikationseinheit 19 ausgestaltet ist. Die kombinierte Einheit "Energiewandler 13 / erste Kommunikationseinheit 19" ist mittels eines drahtlosen Kommunikationskanals 35 mit der zweiten Kommunikationseinheit 33 verbunden. Außerdem sind die Sensoren 15, 16 und 17 mittels Kommunikationsleitungen 28, 29 bzw. 30 mit der kombinierten Einheit "Energiewandler 13 / erste Kommunikationseinheit 19" verbunden.

Die kombinierte Einheit "Energiewandler 13 / erste Kommunikationseinheit 19" kann z.B. eine Spule sein, welche die Energie von einer als Statorspule ausgebildeten Energiequelle 20 aufnimmt. Bei geeigneter Platzierung von Permanentmagneten im Pumpengehäuse 12 kann auch auf die Energiequelle 20 verzichtet werden. Die in der Rotorspule induzierte Wechselspannung wird z.B. über den Akku oder Gleichrichter 18 den Sensoren 15, 16, 17 zur Verfügung gestellt. Die Messsignale der Sensoren 15, 16, 17 werden in den Sensoren 15, 16, 17 in kodierte Spannungssignale umgesetzt und über die Kommunikationsleitungen 28, 29, 30 an die kombinierte Einheit "Energiewandler 13 / erste Kommunikationseinheit 19" weitergeleitet. Alternativ kann hierfür auch mindestens eine gesonderte Kodierungseinheit vorgesehen sein. Durch die kodierten Spannungssignale in der Spule der kombinierten Einheit "Energiewandler 13 / erste Kommunikationseinheit 19"entstehen Magnetfeldvariationen, die wiederum durch als zweite Kommunikationseinheit 33 ausgebildete Spulen am Pumpengehäuse 12 gemessen oder ausgelesen werden können.

Die kombinierte Einheit "Energiewandler 13 / erste Kommunikationseinheit 19" sorgt also nicht nur für die Energieübertragung zwischen der Energiequelle 20 und den Sensoren 15, 16 und 17 sondern ist auch für die Übertragung der Daten oder Signale der Sensoren 15, 15, 17 zu der zweiten Kommunikationseinheit 33 zuständig.

In einer bevorzugten Ausgestaltung unterscheiden sich die Wechselspannungsfrequenzen der Datenübertragung und der Energieübertragung deutlich, d.h. mehr um z.B. 50%, damit es nicht zu unerwünschten Überlagerungen und Störsignalen kommt. Eine für die Datenübertragung genutzte Frequenz sollte weiter z.B. deutlich größer sein als eine mittlere Rotationsfrequenz des Rotors 9, z.B. mindestens 2, 3, 5, 10 oder 50 mal so groß. Andererseits sollte die für die Datenübertragung genutzte Frequenz klein genug sein, um den Effekt des faradayschen Käfigs des (metallischen) Pumpengehäuses 12 gering zu halten.

Bei einer Funkverbindung kommen für die Kommunikation z.B. die ISM-Bänder (Industrial, Scientific and Medical-Bänder bei etwa ∼6.7MHz, ∼13.5MHz, ∼27MHz, ∼40MHz, ∼433MHz, ∼902MHz, ∼2.4GHz, ∼5.8GHz) sowie der Bereich von 120kHz bis 150kHz in Frage.

Ist die Übertragungsstecke komplett abgeschirmt, können auch andere Frequenzen benutzt werden, da die Pumpe als System dann keine hochfrequenten Emissionen erzeugt.

Für die Energieversorgung kommen insbesondere auch niedrige Frequenzen in Frage. Diese können z.B. im Bereich 10Hz bis 100kHz liegen.

Weiter ist sowohl in der Fig. 5 als auch in der Fig. 4 die Energiequelle 20 mittels einer Kommunikationsleitung 34 mit der Steuer- und Verarbeitungseinheit 31 verbunden. Mit anderen Worten steuert die Steuer- und Verarbeitungseinheit 31 auch die Energiequelle 20 entsprechend dem Energiebedarf der Sensoren 15, 16 und 17 an. Weiter ist die Steuer- und Verarbeitungseinheit 31 insbesondere ausgestaltet, Sensorsignale des mindestens einen Sensors 15, 16, 17 auszuwerten und basierend auf den Sensorsignalen die Pumpe 3 bzw. den Rotor 9 anzusteuern.

In einer weiteren Variante umfasst der Rotor 9 einen Sensor 15, welcher als Rotorspule ausgestaltet ist. In dieser Rotorspule induzierte Ströme können dann durch eine am Pumpengehäuse 12 angeordnete Auslesevorrichtung, z.B. die oben genannte Statorspule, ausgelesen werden und an die Steuer- und Verarbeitungseinheit 31 weitergeleitet werden. In dieser Variante kann auf den Energiewandler 13 verzichtet werden.

Das Pumpengehäuse 12 der vorstehenden Ausführungsbeispiele kann ein Kunststoffgehäuse, ein Keramikgehäuse oder ein Metallgehäuse sein. Obwohl die oben beschriebene Pumpe 3 als Axialpumpe ausgestaltet ist, kann die Pumpe 3 auch als Radialpumpe ausgebildet sein. Je nach Ausführung der Pumpe beträgt ein Gewicht des Rotors höchstens 15 g (im Falle einer Axialpumpe), und ein Gewicht des Rotors der Radialpumpe beträgt vorzugsweise höchstens 40 g. Dadurch, dass das Gewicht der Pumpe 3 insgesamt mindestens 100 g und/oder höchstens 250 g beträgt, ist das Gewicht des Rotors 9 viel geringer als das Gewicht der gesamten Pumpe 3.

Mit der Erfindung wird auch ein Verfahren bereitgestellt, das zumindest den folgenden Schritt aufweist: Erfassen von physiologischen Parametern und/oder Strömungs- und/oder Bewegungsparametern durch einen zuvor beschriebenen Sensor 15, 16, 17, der auf dem Rotor 9 oder in dem Rotor 9 angeordnet ist.

Lediglich in den Ausführungsbeispielen beschriebene Merkmale können einzeln beansprucht oder miteinander kombiniert werden.

### Bezugszeichenliste

1. Patientenkörper
2. Herzkammer
3. Blutpumpe
4. Apex
5. Einlassstutzen
6. Ansaugöffnung
7. Auslass
8. Kanüle
9. Rotor
10. Stator
11. Steuervorrichtung
11a. externes Gehäuse
11b. Steuervorrichtung
12. Pumpengehäuse
13. Energiewandler
14. Wendel
15. Sensor
16. Sensor
17. Sensor
18. Akkumulator/Gleichrichter
19. erste Kommunikationseinheit
20. Energiequelle
21. Permanentmagnet
22. Sende- und Empfangsmodul
23. Digital/Analog-Wandler
24a. Nabe
24b. Nabe
25. Versorgungsleitung
26. Versorgungsleitung
27. Versorgungsleitung
28. Kommunikationsleitung
29. Kommunikationsleitung
30. Kommunikationsleitung
31. Steuer- und Verarbeitungseinheit
32. Rotorsteuerung
33. zweite Kommunikationseinheit
34. Kommunikationsleitung
35. Kommunikationskanal
36. Kommunikationskanal
37. Rotationsachse

## Patentansprüche

1. Pumpe (3) zur Förderung von Körperflüssigkeiten, insbesondere Blut, wobei die Pumpe (3) ein Pumpengehäuse (12) und einen im Pumpengehäuse (12) gelagerten Rotor (9) aufweist,
**dadurch gekennzeichnet,**
**dass** der Rotor (9) mindestens einen Sensor (15, 16, 17) zur Erfassung von physiologischen Parametern und/oder Strömungs- und/oder Bewegungsparametern umfasst.

2. Pumpe (3) nach Anspruch 1, **dadurch gekennzeichnet, dass** der Rotor (9) einen Energiewandler (13) zur drahtlosen Energieversorgung des Sensors (15, 16, 17) aufweist.

3. Pumpe (3) nach Anspruch 2, umfassend eine Energiequelle (20) für den Sensor (15, 16, 17), welche ausgestaltet ist, Energie drahtlos an den Energiewandler (13) zu übertragen, wobei die Energiequelle (20) und der Energiewandler (13) derart ausgestaltet sind, dass durch die Energiequelle (20) bereitgestellte Energie im Energiewandler (13) des Rotors (9) in eine für den Betrieb des Sensors (15, 16, 17) notwendige Energieform umgewandelt wird.

4. Pumpe (3) nach Anspruch 2 oder 3, wobei der Rotor (9) einen Energiespeicher (18) aufweist, wobei der Energiespeicher (18) durch in dem Energiewandler (13) erzeugten, elektrischen Strom aufladbar ist.

5. Pumpe (3) nach einem der Ansprüche 2 bis 4, **dadurch gekennzeichnet, dass** der Energiewandler (13) eine Rotorspule, eine Photodiode oder ein piezoelektrischer Rotorwandler ist.

6. Pumpe (3) nach Anspruch 5, wobei das Pumpengehäuse (12) und die Rotorspule derart ausgebildet sind, dass in der Rotorspule bei einer Rotordrehung eine Spannung induzierbar ist.

7. Pumpe (3) nach einem der Ansprüche 5 bis 6, **dadurch gekennzeichnet, dass** das Pumpengehäuse (12) für die Induktion der Spannung in der Rotorspule im Bereich des Rotors (9) mindestens einen Permanentmagneten aufweist.

8. Pumpe (3) nach einem der Ansprüche 5 bis 7, **dadurch gekennzeichnet, dass** das Pumpengehäuse (12) eine Statorspule aufweist, wobei eine vorbestimmte Spannung in der Rotorspule durch eine Bestromung der Statorspule induzierbar ist.

9. Pumpe nach Anspruch 8, **gekennzeichnet durch** eine mit der Statorspule verbundene Steuer- und Verarbeitungsvorrichtung (11, 31), die ausgebildet ist, die Statorspule derart anzusteuern, dass eine vorbestimmte Spannung in der Rotorspule induziert wird.

10. Pumpe (3) nach einem der Ansprüche 5 bis 9, **dadurch gekennzeichnet, dass** die Pumpe (3) einen Schallquelle für eine energetische Kopplung mit dem piezoelektrischen Rotorwandler oder eine Lichtquelle für eine energetische Kopplung mit der Photodiode aufweist.

11. Pumpe (3) nach einem der vorstehenden Ansprüche mit einer Kommunikationseinheit (19) zum drahtlosen Übermitteln von Sensorsignalen des Sensors (15, 16, 17), wobei die Kommunikationseinheit (19) auf oder in dem Rotor (9) angeordnet ist.

12. Pumpe nach den Ansprüchen 2 und 11 mit einer Rotorspule, wobei die Rotorspule sowohl als erste Kommunikationseinheit (19) als auch als Energiewandler (13) ausgebildet ist.

13. Pumpe nach Anspruch 11 oder 12 mit einer Kodierungseinheit, welche dazu ausgebildet ist, Signale der Sensoren (15, 16, 17) zu kodieren und die kodierten Signale an die Kommunikationseinheit (19) weiterzuleiten.

14. Pumpe (3) nach einem der vorstehenden Ansprüche, **dadurch gekennzeichnet, dass** der Sensor (15, 16, 17) ein Beschleunigungssensor, ein Gyroskop, ein Kraftsensor, ein Flusssensor, ein Sauerstoffsensor, ein CO₂-Sensor, ein Näherungssensor, ein Lichtsensor, ein Positionssensor, ein Drucksensor, ein Temperatursensor, ein Magnetfeldsensor, eine Spule, ein RFID-Sensor oder ein pH-Sensor ist.

15. Pumpeneinrichtung, umfassend eine Pumpe (3) nach einem der vorstehenden Ansprüche und eine Steuer- und Verarbeitungseinheit (31), wobei die Steuer- und Verarbeitungseinheit (31) ausgestaltet ist, Sensorsignale des mindestens einen Sensors (15, 16, 17) zu verarbeiten und/oder auszuwerten und basierend auf den Sensorsignalen die Pumpe (3) anzusteuern.

16. Verfahren zum Betreiben einer Pumpe nach einem der Ansprüche 1 bis 14 oder einer Pumpeneinrichtung nach Anspruch 15, mit dem Schritt: Erfassen von physiologischen Parametern und/oder Strömungs- und/oder Bewegungsparametern durch einen Sensor (15, 16, 17), der auf dem Rotor (9) oder in dem Rotor (9) angeordnet ist.
